# EUROPÄISCHE PATENTSCHRIFT

(11) **EP 3 069 129 B1**
(45) Veröffentlichungstag und Bekanntmachung des Hinweises auf die Patenterteilung: **02.09.2020**
(21) Anmeldenummer: 14812405.0
(22) Anmeldetag: 13.11.2014
(51) Int. Cl.: G01N 27/04, G01N 33/38

(54) **SYSTEM UND VERFAHREN ZUR ÜBERWACHUNG EINES UNTERGRUNDES HINSICHTLICH SCHÄDEN UND/ODER ZUM SCHUTZ EINES UNTERGRUNDES VOR SCHÄDEN**
SYSTEM AND METHOD FOR MONITORING A SUBSTRATUM WITH REGARD TO DAMAGE AND/OR FOR PROTECTING A SUBSTRATUM FROM DAMAGE
SYSTÈME ET PROCÉDÉ DE SURVEILLANCE D'UN SOUS-SOL POUR DÉTERMINER SES DOMMAGES ET/OU POUR LA PROTECTION D'UN SOUS-SOL CONTRE DES DOMMAGES

(30) Priorität: 13.11.2013 DE 102013018917
(43) Veröffentlichungstag der Anmeldung: 21.09.2016
(73) Patentinhaber: Rheinisch-Westfälische Technische Hochschule Aachen, 52062 Aachen (DE)
(72) Erfinder: RAUPACH, Michael, 52078 Aachen (DE); REICHLING, Kenji, 10777 Berlin (DE); HELM, Christian, 52062 Aachen (DE)
(74) Vertreter: Cohausz Hannig Borkowski Wißgott
(86) Internationale Anmeldenummer: PCT/EP2014/003040
(87) Internationale Veröffentlichungsnummer: WO 2015/070982

(56) Entgegenhaltungen:
- WO-A-84/01626
- JP-A- H06 257 097
- US-A- 2 668 202
- US-B2- 6 828 808
- MARTINEZ I ET AL: "CORROSION CHARACTERIZATION OF REINFORCED CONCRETE SLABS WITH DIFFERENT DEVICES", CORROSION, NACE INTERNATIONAL, HOUSTON, TX; US, US, Bd. 64, Nr. 2, 1. Februar 2008 (2008-02-01), Seiten 107-123, XP001511551, ISSN: 0010-9312

## Beschreibung

Die Erfindung betrifft ein System zur Überwachung eines Untergrundes hinsichtlich Schäden und/oder zum Schutz eines Untergrundes vor Schäden entsprechend dem unabhängigen Anspruch 1. Die Erfindung betrifft weiterhin ein Verfahren zur Überwachung beziehungsweise auch zum Schutz eines Untergrundes hinsichtlich Schäden entsprechend dem unabhängigen Anspruch 15 sowie 16, sowie ein Verfahren zur Herstellung eines eingangs genannten Systems entsprechend dem unabhängigen Anspruch 12.

Im Stand der Technik ist es bekannt, dass Untergründe, z.B.

Untergrundkonstruktionen, beispielsweise solche aus stahlbewehrtem Beton, Schäden erleiden können, z.B. wenn diese Feuchtigkeit ausgesetzt sind, insbesondere durch chemische Elemente, die mit der Feuchtigkeit in den Untergrund eingetragen werden. Schäden können somit zum Beispiel entstehen, wenn die Feuchtigkeit in die Untergrundkonstruktion eindringt und eine darin vorhandene Stahlbewehrung hierdurch bedingt zu korrodieren beginnt, insbesondere wenn durch die Feuchtigkeit Salze, insbesondere Chloridionen solcher Salze in diese Untergrundkonstruktion eingetragen werden. Dies kann beispielsweise erfolgen durch eine Tausalzbelastung im Winter, insbesondere bei Fahrbahnen, die zu solchen Untergrundkonstruktionen zählen.

Untergründe im Sinne der Erfindung können neben den hier exemplarisch genannten Fahrbahnen, insbesondere fahrbahnbildende Brückenkonstruktionen sein, sowie auch sonstige andere, insbesondere statisch belastete Bauwerke, besonders solche aus Beton und insbesondere solche aus stahlbewehrtem Beton oder auch aus anderen zementgebundenen, insbesondere hydraulisch härtenden Massen.

Die Erfindung ist jedoch nicht auf Untergrundkonstruktionen aus stahlbewehrtem Beton beschränkt. Auch Untergrundkonstruktionen rein aus Metall, beispielsweise Stahlträger oder auch reines Erdreich, z.B, unterhalb von Deponien können und sollen mit einem erfindungsgemäßen System überwacht und gegebenenfalls auch vor Schäden, z.B. Feuchteschäden oder Schäden durch chemische Verunreinigung geschützt werden.

Bisherige Systeme zur Überwachung und/oder zum Schutz von Untergründen hinsichtlich Schäden, wie z.B. Feuchteschäden sehen vor, eine Abdichtungen eines solchen Untergrundes, insbesondere über einem Untergrund vorzunehmen, um zu verhindern, dass Feuchtigkeit und ggfs. damit transportierte Stoffe in diese Untergründe eindringen kann. Solche Abdichtungen werden zum Teil nicht mit der entsprechenden Sorgfalt durchgeführt beziehungsweise können im Laufe der Zeit altern und hierdurch undicht werden und somit zeitlich verzögert Schäden an einem Untergrund zulassen.

Unter einem Schaden im Sinne der Erfindung wird nicht nur ein durch Feuchte entstandener Schaden angesehen, sondern auch schon das Eintreten von Feuchtigkeit selbst, z.B. aufgrund eines Schadens in einer Abdichtung.

Bisherige Systeme zur Überwachung von Untergründen hinsichtlich Schäden, wie z.B. Feuchteschäden sehen es beispielsweise vor, mit mobilen Vorrichtungen die Oberfläche eines Untergrundes, z.B. die Betonoberfläche einer aus stahlbewehrtem Beton hergestellten Untergrundkonstruktion abzufahren, hierbei Elektroden mit der Untergrundkonstruktion in Kontakt zu bringen und Spannungspotentiale zu messen, um so Korrosionsstellen in der Stahlbewehrung einer Untergrundkonstruktion ausfindig zu machen. Ersichtlich sind solche Systeme aufwendig, personalintensiv und können nur auf Anforderung eine Überwachung vornehmen, nicht jedoch eine dauerhafte Überwachungsmöglichkeit bieten. Darüber hinaus können solche Systeme auf beschichteten Untergründen nicht zum Einsatz kommen.

Auch haben solche Systeme den Nachteil, dass nur bereits aufgetretene Schäden erkannt werden können, ein Ereignis, das zu einem Folgeschaden führen wird, wie z.B. ein Auftreten von Feuchtigkeit z.B. bei schadhafter Abdichtung jedoch nicht vor einer weiteren Schadensentstehung feststellbar ist.

US 6 828 808 offenbart Elektroden zur Bestimmung der Leitfähigkeit die an einem Medium z.B. Bauteil aus Beton angebracht werden zum Detektieren von Korrosion.

WO84/01626 offenbart eine Detektion von Flüssigkeiten in Sanitärprodukten mittels einer elektrolytisch wirksame Schicht mit einem feuchtigkeitsabhängigen elektrischen Widerstand und daran angebrachten Elektroden aus elektrisch leitfähigen Kunststoff mit Durchgängen in den Elektroden.

Es ist daher eine Aufgabe der Erfindung, ein System zur Überwachung eines Untergrundes hinsichtlich Schäden, besonders hinsichtlich Feuchteschäden und/oder zum Schutz einer Untergrundkonstruktion vor Schäden, besonders Feuchteschäden bereitzustellen, das insbesondere ohne personellen Einsatz, weiter bevorzugt dauerhaft und darüber hinaus bevorzugt großflächig bei beliebigen Untergründen und bevorzugt bei Untergrundkonstruktionen, wie zum Beispiel solche von Fahrbahnen, Brücken oder sonstigen Bauwerken, zum Einsatz kommen kann. Es ist weiterhin eine Aufgabe, ein solches System bereitzustellen, das bevorzugt Teil eines Gesamtbauwerkes in Verbindung mit dem Untergrund, bzw. der Untergrundkonstruktion wird, somit also dauerhaft mit dem Untergrund/der Untergrundkonstruktion verbunden ist und zusammen mit diesem / dieser einer Nutzung zugeführt wird.

Weiterhin ist es eine Aufgabe in Verbindung mit einem solchen System ein Verfahren zur dauerhaften großflächigen Überwachung beziehungsweise zum Schutz eines Untergrundes bereitzustellen, sowie auch ein wirtschaftlich und zuverlässig durchführbares Herstellungsverfahren bereitzustellen.

Diese Aufgabe wird erfindungsgemäß durch ein System der eingangs genannten Art gelöst, bei dem es vorgesehen ist, dass dieses System eine elektrolytisch wirksame Schicht umfasst, die einen feuchtigkeitsabhängigen elektrischen Widerstand aufweist und es wenigstens ein Elektrodenpaar aufweist, deren beabstandete Elektroden über die elektrolytisch wirksame Schicht miteinander verbunden, insbesondere elektrisch verbunden sind und das System weiterhin eine Messvorrichtung umfasst, mittels welcher eine Eigenschaft, insbesondere eine elektrische Größe der elektrolytisch wirksamen Schicht oder der Elektroden mithilfe von Elektroden wenigstens eines, insbesondere jedes Elektrodenpaares messbar ist und/oder eine Steuervorrichtung umfasst, mittels der eine elektrische Spannung an die Elektroden wenigstens eines Elektrodenpaares anlegbar ist und wenigstens eine der Elektroden des wenigstens einen Elektrodenpaares als eine flächige Elektrode in der elektrolytisch wirksamen Schicht ausgebildet ist, insbesondere deren Fläche parallel zur Oberfläche des Untergrundes orientiert ist.

Unter einer elektrolytisch wirksamen Schicht wird hier eine Schicht verstanden, die entweder bereits ursprünglich, d.h. auch ohne Schaden verursachendes Ereignis, zumindest aber nach einem Schaden verursachenden Ereignis, wie z.B. einem Feuchtigkeitseintritt in die Schicht, einen Elektrolyten ausbildet. Die Schicht muss also zumindest so ausgebildet sein, dass eine lonenwanderung in der Schicht möglich ist, z.B. nachdem sie feucht geworden ist. Zumindest mit Eintritt eines Schaden verursachenden Ereignisses, wie Feuchtigkeitseintritt wird somit die Schicht elektrisch leitfähig, sofern Sie es nicht auch bereits ohne das Ereignis ist.

Ein System der erfindungsgemäßen Art umfasst demnach immer wenigstens ein Elektrodenpaar aus zwei Elektroden, die über die elektrisch leitfähige und elektrolytisch wirksame Schicht miteinander elektrisch verbunden sind, so dass mittels dieser beiden Elektroden des wenigstens einen Elektrodenpaares eine Eigenschaft dieser Schicht, bevorzugt eine elektrische Größe der Schicht über eine Messvorrichtung ermittelt werden kann und anhand der gemessenen Größe, insbesondere des konkreten Wertes dieser Größe ein Rückschluss gezogen werden, ob ein Schaden verursachendes Ereignis stattgefunden hat.

Grundsätzlich kann die Erfindung vorsehen, jegliche Art von Eigenschaft, insbesondere elektrischer Eigenschaft der Schicht durch das Messen einer elektrischen messbaren Größe zu überwachen und aus einer Änderung des Messwertes oder einem Herausfallen des Messwertes aus einem Toleranzintervall oder bei Überschreiben bzw. Unterschreiten eines für einen Vergleich herangezogenen Vergleichswertes auf einen Schadensereignis zu schließen und dieses zu signalisieren. Eine überwachende Messung kann zeitlich wiederholend stattfinden, so dass auch eine zeitliche Entwicklung beobachtbar und auswertbar ist.

Eine Ausführung kann z.B. vorsehen, dass der elektrische Widerstand der elektrolytisch wirksamen Schicht als Eigenschaft überwacht wird.

Es kann demnach im Rahmen eines Verfahrens zur Überwachung eines Untergrundes vorgesehen sein, den Widerstand zwischen den Elektroden zu messen und mit einem vorgegebenen Widerstands-Vergleichswert zu vergleichen, wobei bei einem Unterschreiten des Vergleichswertes aufgrund ansteigender Feuchtigkeit in der Schicht zwischen den wenigstens zwei Elektroden, dieses Unterschreiten als ein Ereignis signalisiert werden kann.

Eine Signalisierung kann allgemein zum Beispiel durch Fernwirkungssignale, wie eine Funkübertragung vorgenommen werden, so dass Wartungspersonal, zum Beispiel in einer entfernten Leitstelle, darüber informiert werden kann, dass ein Schaden verursachendes Ereignis, z.B. ein Feuchteeintritt in einem solchen Bereich gegeben ist, der durch die Flächengröße der flächig ausgebildeten Elektrode überwacht wird.

Hier kann es beispielsweise vorgesehen sein, nach Fertigstellung eines Untergrundes oder einer Untergrundkonstruktion und Anbringung eines erfindungsgemäßen Systems auf dieser, einen Messwert für die überwachte Eigenschaft, z.B. also den Widerstand zwischen den Elektroden zu erfassen, der einen schadenfreien Referenzzustand, insbesondere also einen trockenen Referenzzustand widerspiegelt und somit zukünftig als Vergleichswert für Messungen herangezogen werden kann, um einen zukünftigen Schadeneintritt, z.B. also einen Feuchteeintritt zwischen die Elektroden anhand des Unterschreitens oder Überschreitens eines Vergleichswertes, z.B. des Vergleichs-Widerstandsmesswertes anzuzeigen beziehungsweise zu signalisieren.

Statt oder zusätzlich zur beispielshaften Überwachung des Widerstandswertes kann es in anderen Ausführungen auch vorgesehen sein, z.B. die elektrische Impedanz oder eine Spannung, die zwischen zwei Elektroden anliegt oder einen Strom, der über die Elektroden und die elektrolytisch wirksame Schicht fließt oder die Dielektrizität der elektrolytisch wirksamen Schicht oder die chemische Zusammensetzung der elektrolytisch wirksamen Schicht, insbesondere des Elektrolyten dieser Schicht oder den pH-Wert der elektrolytisch wirksamen Schicht oder die Temperatur der elektrolytisch wirksamen Schicht überwachend zu messen, insbesondere nicht mehr tolerable Abweichungen zu signalisieren. Es können auch Größen überwacht werden, die nicht direkt gemessen werden, die sich aber aus mehreren anderen Messungen ergeben. Insbesondere kann auch eine Eigenschaft der Elektroden selbst gemessen werden, z.B. über die Elektrodenpolarisation. Hierfür können Elektrodenmaterialien gewählt werden, die gegenüber der gewünschten zu überwachenden Eigenschaft eine Sensitivität, insbesondere ausreichend große Sensitivität aufweisen.

Geeignete Untergründe, bei denen das System oder auch ein Verfahren zur Überwachung zum Einsatz kommen können sind in nicht abschließender Aufzählung z.B. Erdreich, eine Deponie, eine Betonunterkonstruktion, insbesondere eine stahlbewehrte Unterkonstruktion, bevorzugt eine Straße oder Brücke, bzw. allgemein Untergründe aus zementgebundenen Massen, insbesondere mit metallischer Bewehrung.

Die elektrolytisch wirksame Schicht entsprechend der Erfindung ist durch eine zementöse ausgehärtete, insbesondere hydraulisch, latent-hydraulisch oder puzzolanisch ausgehärtete Schicht, wie z.B. ein Mörtel oder Beton oder durch ein offenporiges elektrolytfreies Gefüge, insbesondere einen offenporigen elektrolytfreien Schaum, das/der in seinen offenen Poren einen Elektrolyten bei einem Schadenereignis aufnehmen kann, insbesondere hierdurch leitfähig wird oder durch ein offenporiges elektrolytgefülltes Gefüge, insbesondere einen offenporigen elektrolytgefüllten Schaum, insbesondere in dem sich die Elektrolytzusammensetzung bei einem Schadenereignis ändert oder z.B. auch durch ein Gel, insbesondere dessen Feuchtegehalt, z.B. an Wasser änderbar ist, ausgebildet.

Ein Verfahren zum Schutz eines Untergrundes vor Feuchteschäden ist erfindungsgemäß vorsehen, dass zwischen wenigstens zwei Elektroden eines Systems der vorgenannten beschriebenen Art mittels einer Steuervorrichtung eine Spannung angelegt wird. Dies kann z.B. erfolgen zum Zweck der Erzeugung einer Diffusionsbarriere.

So wird durch das Anlegen einer Spannung zwischen zwei Elektroden ein elektrisches Feld mit einer durch das Vorzeichen der Spannungsdifferenz zwischen den Elektroden gegebenen Feldrichtung erzeugt, welches als Barriere dienen kann für Ionen, zum Beispiel Chloridionen, so dass durch ein solches Verfahren, insbesondere nach Feststellen eines Feuchteereignisses, zumindest das Eindringen schädigender Bestandteile, wie zum Beispiel von Chloridionen oder auch von anderen Ionen, wirksam durch eine derart aufgebaute Diffusionsbarriere verhindert werden kann.

Auch kann durch das Anlegen einer Spannung ein kathodischer Korrosionsschutz erzielt werden.

Ein solches Verfahren zum Schutz eines Untergrundes bzw. einer Untergrundkonstruktion kann unabhängig von dem zuvor benannten Verfahren zur Überwachung eines Untergrundes bzw. einer Untergrundkonstruktion eingesetzt werden, in besonderer Ausführungsgestaltung jedoch ergänzend zu diesem, nämlich insbesondere dann, wenn zuvor im Rahmen der Überwachung ein Schaden verursachendes Ereignis, z.B. ein Feuchteeintritt in das erfindungsgemäße System festgestellt wurde.

Beispielsweise kann es hierfür vorgesehen sein, statt einer zuvor zum Zwecke der Überprüfung, insbesondere einer Widerstandsmessung vorgesehenen Messvorrichtung, nach Feststellen eines Schadensereignisses, insbesondere durch eingetretene Feuchtigkeit zwischen das überwachte Elektrodenpaar bzw. in die elektrolytisch wirksame Schicht, die Messvorrichtung von dem Elektrodenpaar abzuschalten und stattdessen die Steuervorrichtung an das Elektrodenpaar anzuschalten, um die Elektroden mit einer Spannung zu beaufschlagen, insbesondere um sodann die Diffusionsbarriere und/oder einen kathodischen Korrosionsschutz aufzubauen.

Insbesondere ein Zeitpunkt für eine nach dem Schadensereignis anstehende Wartung kann demnach besser überbrückt werden, ohne dass es in der Zwischenzeit zu signifikanten Schädigungen im Untergrund bzw. in der Untergrundkonstruktion kommen kann, da in der Zwischenzeit ein Korrosionsschutz erzielt wird.

Eine Ausführungsvariante des erfindungsgemäßen Systems kann es hier vorsehen, dass ein solches System direkt auf einem Untergrund, bzw. einer Untergrundkonstruktion, insbesondere einer stahlbewehrten Betonuntergrundkonstruktion befestigt ist bzw. verfahrensgemäß gefestigt wird.

Hierfür kann beispielsweise die elektrolytisch wirksame Schicht bzw. das Material zum Einsatz kommen, das auch diese Schicht ausbildet, in welcher wenigstens eine Elektrode des wenigstens einen vorhandenen Elektrodenpaares des System angeordnet ist. Besonders geeignet sind hierfür klebende Schichtmaterialien, wie z.B. die eingangs genannten zementgebundenen Massen, insbesondere Mörtel oder Beton oder schäumende Kleber.

Ein solches Material bzw. die daraus hergestellte Schicht kann demnach nicht nur die Funktion einer elektrolytisch leitfähigen Umgebung um diese wenigstens eine Elektrode übernehmen, sondern auch eine Befestigungsfunktion, um eine kraft- und/oder formschlüssige Verbindung mit dem Untergrund bzw. einer Untergrundkonstruktion zu erzielen.

Eine Befestigung muss vorliegend jedoch nicht zwingend erfolgen. Es besteht auch die Möglichkeit, ein erfindungsgemäßes System nur auf einen Untergrund aufzulegen oder eine Haftung nur durch Adhäsion zu erzielen. So kann auch ggfs. eine spätere rückstandslose Entfernung eines solchen Systems erfolgen.

Eine Ausführungsvariante des Systems kann es hierbei vorsehen, dass eine erste Elektrode eines Elektrodenpaares durch die flächige Elektrode in der elektrolytisch wirksamen Schicht, z.B. der Mörtelschicht und eine zweite Elektrode eines Elektrodenpaares durch den Untergrund selbst (sofern dieser leitfähig ist) oder ein darin befindliches elektrisch leitfähiges Elementausgebildet ist, wie beispielsweise durch eine metallische Bewehrung der Untergrundkonstruktion gebildet ist, insbesondere wenn der Untergrund durch stahlbewehrten Beton gebildet wird..

Diese Ausbildung des Systems hat den Vorteil, dass eine der beiden Elektroden des wenigstens einen vorhandenen Elektrodenpaares direkt bereits durch Elemente des Untergrundes bzw. der Unterkonstruktion gebildet wird, nämlich beispielsweise durch Bewehrungselemente, insbesondere Bewehrungsstäbe oder Bewehrungsgitter. So kann demnach eine eingangs genannte Messvorrichtung beziehungsweise eine Steuervorrichtung sowohl an der metallischen Bewehrung der Unterkonstruktion als auch an einer in der Mörtelschicht angeordneten flächigen Elektrode angeschlossen werden und so die eingangs beschriebenen Funktionen wahrnehmen.

Ein derartiges System zur Überwachung beziehungsweise zum Schutz eines Untergrundes bzw. einer Untergrundkonstruktion kann demnach besonders kostengünstig ausgefertigt werden, da es lediglich der Anbringung einer weiteren Elektrode beziehungsweise mehreren flächigen nebeneinander angeordneten Elektroden oberhalb der Bewährungselemente bedarf.

Gegebenenfalls als nachteilig wird bei einer solchen Konstruktion des Systems empfunden, dass festgestellte Feuchtigkeit in den Bereich zwischen einer flächigen Elektrode und dem Untergrund oder den Bewährungselementen und somit bereits in den Untergrund / die Untergrundkonstruktion eingedrungen ist und somit bereits bei Signalisierung eines Feuchteeintrags unmittelbar auch Schädigungen der Untergrundkonstruktion auftreten können. Allgemein wird also erst das bereits stattgefundene Eintreten einer Schädigung des Untergrundes bzw. der Untergrundkonstruktion detektiert.

Eine erfindungsgemäß bevorzugte Weiterbildung des Systems kann es demnach vorsehen, dass eine erste Elektrode des Elektrodenpaares durch die flächige Elektrode in der elektrolytisch wirksamen Schicht, z.B. der Mörtelschicht und eine zweite Elektrode eines Elektrodenpaares durch eine weitere, insbesondere ebenfalls flächige Elektrode in derselben elektrolytische wirksamen Schicht, insbesondere Mörtelschicht ausgebildet ist, wobei besonders bevorzugt beide Elektroden in der Mörtelschicht flächengleich und formgleich ausgebildet sind und in einer Abstandsrichtung fluchtend hintereinander und weiter bevorzugt parallel hintereinander liegen.

So kann bei einer solchen Ausfertigung des Systems das Gesamtsystem für sich autark realisiert werden, ohne den Untergrund oder Elemente der zu schützenden beziehungsweise zu überwachenden Untergrundkonstruktion in das System mit einzubinden.

Darüber hinaus hat diese Ausfertigung des Systems den Vorteil, dass das Unter-/Überschreiten wenigstens eines Vergleichswertes bei der Messung einer Eigenschaft, wie z.B. des Widerstandes zwischen zwei Elektroden eines derartig in der Schicht ausgebildeten Elektrodenpaares anzeigt, dass ein Schaden über dem Untergrund, aber noch nicht im Untergrund vorliegt, also z.B. Feuchtigkeit zwischen die beiden Elektroden des überwachten Elektrodenpaares eingedrungen ist, jedoch noch nicht in den Untergrund bzw. die Untergrundkonstruktion selbst, da erfindungsgemäß das System zur Überwachung beziehungsweise zum Schutz eines Untergrundes / einer Untergrundkonstruktion in Schwerkraftrichtung über dem Untergrund bzw. der zu überwachenden beziehungsweise zu schützenden Untergrundkonstruktion angeordnet wird.

Beispielsweise Feuchtigkeit ist demnach bei erstmaligem messtechnischem Feststellen zwar bis in einen Bereich zwischen die Elektroden vorgedrungen, jedoch noch nicht bis in die Untergrundkonstruktion selbst.

Insbesondere in diesem Fall ist es empfehlenswert, das Verfahren zur Überwachung eines Untergrundes / einer Untergrundkonstruktion zu kombinieren mit dem bereits zuvor beschriebenen Verfahren zum Schutz eines Untergrundes / einer Untergrundkonstruktion, um nämlich sodann nach Feststellung eines Schaden verursachenden Ereignisses, wie z.B. eines Feuchtigkeitseinbruches in dem System den darunter liegenden Untergrund / die darunterliegende Untergrundkonstruktion zu schützen durch Anschalten einer Spannung an die überwachten Elektroden des Elektrodenpaares oder aber auch an andere Elektroden innerhalb des erfindungsgemäßen Systems, um die bereits eingangs beschriebene Diffusionsbarriere gegen Ionen und/oder einen kathodischen Korrosionsschutz auszubilden und so eine schädigende Einflussnahme auf den Untergrund oder Elemente in der Untergrundkonstruktion zu verhindern.

Wenngleich die Erfindung hier im Rahmen der Beschreibung des Systems im Wesentlichen in einer Realisation mit zwei Elektroden und somit einem Elektrodenpaar innerhalb der elektrolytisch wirksamen Schicht, z.B. Mörtelschicht des Systems bevorzugt beschrieben wird, kann eine Ausfertigung der Erfindung auch vorsehen, dass das System mehr als zwei Elektroden der zuvor beschriebenen Art innerhalb der Schicht, z.B. Mörtelschicht aufweist, so dass in vertikaler Richtung über einem Untergrund oder einer Untergrundkonstruktion auch ortliche Profile der überwachten Eigenschaft, z.B. Feuchtigkeitsprofile erfasst werden können, beispielsweise durch Messung der elektrischen Widerstände zwischen Elektrodenpaaren in unterschiedlicher Höhe der Schicht, insbesondere der bereits beschriebenen Mörtelschicht.

Insbesondere kann auch beim Einsatz von mehr als zwei Elektroden und besonders den zuvor beschriebenen flächig ausgebildeten Elektroden ebenso auch der Untergrund oder die Untergrundkonstruktion selbst mit in das System eingebunden werden, indem nämlich auch hier Elemente des Untergrundes, z.B. die Bewehrung als eine weitere Elektrode zur Bildung eines Elektrodenpaares mit einer solchen Elektrode innerhalb der elektrolytisch wirksamen Schicht, bevorzugt des Mörtels verwendet wird.

Z.B. kann, beispielsweise auch dauerhaft zwischen der Bewehrung und einer darüber liegenden Elektrode in der Schicht eine Spannung zur Erzeugung einer Diffusionsbarriere oder eines kathodischen Korrosionsschutzes erzeugt werden, wobei zwischen einem darüber liegenden Elektrodenpaar eines bestimmte Eigenschaft, z.B. der Widerstand gemessen wird.

Die eingangs beschriebenen flächigen Elektroden, von denen wenigstens eine innerhalb einer elektrolytisch wirksamen Schicht, z.B. einer Mörtelschicht oberhalb der zu schützenden beziehungsweise zur überwachenden Untergrundkonstruktion vorhanden ist, bevorzugt aber wenigstens zwei innerhalb der Schicht, z.B. der Mörtelschicht angeordnet sind, können beispielsweise ausgebildet sein, als eine Schicht von elektrisch leitfähigen Fasern, die in einem Matrixmaterial eingebunden sind, also z.B. in Mörtel oder Beton.

Als Faser können hier besonders bevorzugt Kohlenstofffasern zum Einsatz kommen, da diese selbst leitfähig, jedoch nicht selbst korrodierend sind und somit besondere Vorteile in Verbindung mit der Erfindung bieten. Beispielsweise kann eine solche Schicht aus elektrisch leitfähigen Fasern dadurch ausgebildet werden, dass das Matrixmaterial, wie z.B. Mörtel oder Beton mit den elektrisch leitfähigen Fasern vermischt wird und eine solche Mischung flächig entweder unmittelbar auf dem Untergrund / der Untergrundkonstruktion oder zunächst auf einer darauf angebrachten Matrixmaterialschicht ohne Fasern z.B. Mörtelschicht ohne Fasern aufgebracht wird, so dass im Endeffekt nach Erstellung des erfindungsgemäßen Systems wenigstens eine solche Schicht, bevorzugt zwei solche Schichten, innerhalb der bereits zuvor genannten elektrisch leitfähigen und elektrolytisch wirksamen Schicht mit feuchtevariablem elektrischen Widerstand angeordnet sind. Das vorgenannte Matrixmaterial, das als Mischung mit Fasern eine flächige Elektrode bilden kann, kann somit bevorzugt ohne Faseranteil auch das Material der elektrolytisch wirksamen Schicht bilden.

Bevorzugt ist sicherzustellen, dass die Matrixmaterialmischung mit Fasern eine höhere elektrische Leitfähigkeit und somit einen geringeren elektrischen Widerstand aufweist als das Material, insbesondere dasselbe Matrixmaterial elektrolytisch wirksamen Schicht, in welche diese elektrisch leitfähige Schicht eingebettet ist.

Eine dem gegenüber bevorzugte Ausführungsform kann es hier vorsehen, eine flächige Elektrode durch ein flächiges leitfähiges Elektrodenelement mit in der Oberfläche des Elektrodenelementes angeordneten Durchgängen auszubilden.

Beispielsweise kann ein solches Elektrodenelement durch ein metallisches oder aber auch durch ein nicht-metallisches, jedoch zumindest leitfähiges, gelochtes Elektrodenelement ausgebildet sein, wobei die Lochung nicht zwingend einen kreisförmigen sondern gegebenenfalls auch eckigen Querschnitt aufweisen kann. Z.B ein Lochblech oder ein Drahtgitter kann diesen Zweck erfüllen.

Erfindungsgemäß ist es vorsehen, dass ein solches Elektrodenelement als eine textile Mattenware oder textile Maschenware aus elektrisch leitfähigen Fasern, insbesondere ein Gewebe aus leitfähigen Fasern ausgebildet ist, insbesondere wobei die in der Maschenware gebildeten Maschen den zuvor benannten Durchgängen entsprechen.

Erfindungsgemäß weisen die Durchgänge beziehungsweise die Maschen einen Querschnitt auf, der größer ist als die größten Feststoffanteile im Schichtmaterial der elektrolytisch wirksamen Schicht, also z.B. größer als das Größtkorn des Mörtels oder Beton, der diese Schicht ausbildet, in der ein solches Elektrodenelement beziehungsweise ein Paar aus zwei solcher Elektrodenelemente aufgenommen ist. Hierdurch wird nämlich erzielt, dass sich diese Feststoffanteile gut durch die Durchgänge hindurch erstrecken können und somit die Elektrodenelemente nicht schichtlösend in der Schicht, z.B. im Mörtel / Beton wirken können.

Bei der Ausbildung eines Elektrodenpaares aus einer solchen, insbesondere textilen Maschenware oder Mattenware kann es vorgesehen sein, demnach die jeweilige Maschenware / Mattenware, die eine Elektrode eines solchen Paares bildet, in der Schicht (z.B. dem Mörtel) und zur anderen Elektrode durch das Schichtmaterial (den Mörtel) beabstandet einzubringen. So können innerhalb der elektrolytisch wirksamen Schicht wenigstens zwei in vertikaler Richtung übereinander liegende flächige Elektroden aus der genannten Maschenware / Mattenware angeordnet werden, um zwischen diesen Elektroden die gewünschte Eigenschaft, z.B. den Widerstand zu messen beziehungsweise zum Schutz eine Spannung anzulegen.

Besonders vorteilhaft wird bei der Anwendung eines Elektrodenpaares innerhalb der genannten elektrolytisch wirksamen Schicht eine Ausführungsform angesehen, bei der das Elektrodenpaar aus zwei Lagen einer Mattenware oder Maschenware aus leitfähigen Fasern bereits als Einheit, insbesondere vorkonfektionierte Einheit auf Abstand gehalten ist. Dafür kann es vorgesehen sein, dass die beiden Lagen der Mattenwaren / Maschenwaren durch ein Gewirk nicht leitender Fasern, insbesondere aus Kunststofffasern, auf Abstand gehalten sind. Das Gewirk kann dabei bevorzugt jeweils auch die Fasern der Mattenware / Maschenware umschlingen, so dass die beiden Maschenwaren / Mattenwaren und das Gewirk eine bauliche Einheit bilden, insbesondere ein solche, die auch aufwickelbar ist.

Die Anordnung eines Elektrodenpaares kann bei dieser Ausfertigung sehr gut als vorkonfektioniertes Bauteil bereitgehalten werden, um dieses auf einem Untergrund bzw. einer Untergrundkonstruktion auszulegen, entweder unmittelbar auf die Untergrundkonstruktion oder in eine zunächst darauf ausgebrachte Verbindungsschicht, insbesondere Mörtelschicht oder Betonschicht.

Das Elektrodenpaar kann sodann in die Verbindungsschicht, z.B. Mörtelschicht oder Betonschicht eingebettet werden, durch weiteren Auftrag von Schichtmaterial, z.B. von Mörtel oder Beton, das/der sich durch die Maschen / Durchgänge hindurch und somit durch das Elektrodenpaar verteilt, so dass im Endeffekt ein erfindungsgemäßes System entsteht, bei dem ein Elektrodenpaar aus mit dem Abstandsgewirk auf Abstand gehaltenen Maschenwaren / Mattenwaren in der elektrolytisch leitfähigen Schicht, insbesondere Mörtelschicht oder Betonschicht angeordnet ist.

Eine andere Alternative kann auch vorsehen als flächige Elektrode ein nichtleitendes Trägerelement elektrisch leitfähig zu beschichten.

Ein erfindungsgemäß bevorzugt eingesetztes Verfahren zur Herstellung eines Systems zur Überwachung und/oder zum Schützen eines Untergrundes / einer Untergrundkonstruktion kann es demnach allgemein vorsehen, dass wenigstens ein flächiges, insbesondere vorkonfektioniertes Elektrodenelement oder wenigstens ein Paar aus zwei flächigen, insbesondere vorkonfektionierten und beabstandeten Elektrodenelementen, die eine Bauteileinheit bilden, insbesondere wobei ein jedes Elektrodenelement eine Maschenware oder Mattenware aus leitfähigen Fasern umfasst, auf einem Untergrund bzw. einer Untergrundkonstruktion, wie beispielsweise einer Fahrbahn, ausgelegt werden, wobei ein solches Auslegen zum Beispiel durch ein Abrollen von einem Wickel erfolgen kann.

Da solche insbesondere vorkonfektionierten Elektrodenelemente beziehungsweise Elektrodenpaare nur in einer bestimmten maximalen Flächengröße hergestellt werden können, kann es die Erfindung weiterhin vorsehen, dass das Auslegen wenigstens eines solchen flächigen insbesondere vorkonfektionierten Elektrodenelementes beziehungsweise insbesondere vorkonfektionierten Paares aus zwei Elektrodenelementen mehrfach in wenigstens einer Richtung wiederholt nebeneinander, insbesondere in Erstreckungsrichtung des Untergrundes der Untergrundkonstruktion erfolgt, um so eine große Fläche des Untergrundes / der Untergrundkonstruktion überwachen beziehungsweise schützen zu können, wobei durch jedes insbesondere vorkonfektionierte Elektrodenelement beziehungsweise Paar von zwei Elektrodenelementen, ein überwachter beziehungsweise schützbarer Flächenbereich definiert wird.

Z.B. bei der Anwendung im Straßenbau / Brückenbau kann es vorgesehen sein, dass Elektrodenelemente oder vorkonfektionierte Paare aus zwei Elektrodenelementen, insbesondere solche die bereits durch Abstandselemente miteinander auf Abstand verbunden sind, in einer Fertigungslänge bereitgestellt werden, die der Breite einer Fahrbahn oder auch der Breite zweier oder auch mehrere Fahrbahnen, insbesondere auch der Breite von einer oder mehrerer Hin- und Rückfahrbahnen entsprechen. Quer zur Fahrbahnrichtung können sodann die Elektrodenelemente jeweils die gesamte Straßenbreite oder zumindest ganze Fahrbahnteile hiervon überdecken und in Straßenerstreckungsrichtung mehrfach nebeneinander gelegt werden.

Durch die Einbettung eines solchen, insbesondere vorkonfektionierten Elektrodenelementes beziehungsweise Paares aus zwei, insbesondere bereits vorkonfektioniert mit Abstandsgewirk verbundenen Elektrodenelementen, kann weiterhin auch zeitgleich eine Befestigung an einem Untergrund / einer Untergrundkonstruktion erfolgen, nämlich mit demselben Schichtmaterial, insbesondere Mörtel oder Beton, das auch für die Einbettung der wenigstens einen flächigen Elektrode in die elektrolytisch wirksame Schicht Verwendung findet.

Jede Elektrode beziehungsweise jedes Elektrodenpaar kann erfindungsgemäß mit einer Messvorrichtung zum Messen einer Eigenschaft, insbesondere elektrischen Eigenschaft und bevorzugt des elektrischen Widerstandes zwischen zwei Elektroden und/oder mit einer Steuervorrichtung zum Anlegen einer Spannung zwischen zwei Elektroden verbunden werden, um die Überwachung und/oder den Schutz desjenigen Flächenbereiches zu erzielen, der durch die Flächengröße eines Elektrodenelementes beziehungsweise insbesondere vorkonfektionierten Paares eines Elektrodenelementes beziehungsweise insbesondere vorkonfektionierten Paares abgedeckt wird.

Ein Ausführungsbeispiel der Erfindung wird anhand der nachfolgenden Figur näher beschrieben.

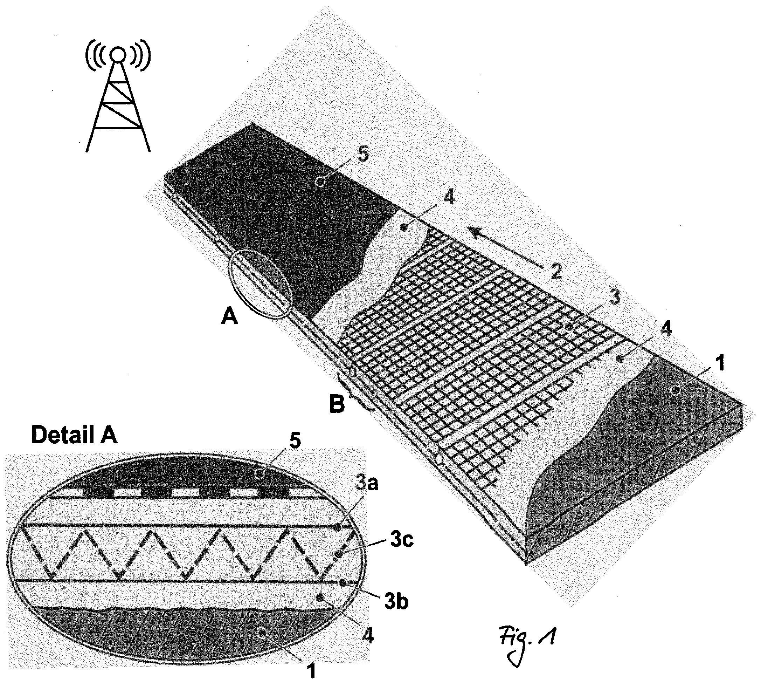

Die Figur 1 zeigt als Beispiel einer Anwendung das System in Verbindung mit einer Brücke 1 beziehungsweise allgemein mit einer Fahrbahn 1 als zu schützende Untergrundkonstruktion.

In der Längserstreckungsrichtung beziehungsweise Fahrrichtung 2 sind hier über die Breite der Fahrbahn hinweg in dieser Ausfertigung vorkonfektionierte, auf Abstand gehaltene Elektrodenpaare 3 angeordnet, wobei jedes Elektrodenpaar 3 gemäß der Detailansicht eine obere Maschenware 3a und eine untere Maschenware 3b umfasst, wobei beide Maschenwaren 3a und 3b durch ein Abstandsgewirk 3c auf einen gewünschten Abstand gehalten sind.

Das Abstandsgewirk ist hier in dieser Ausfertigung aus nicht-leitenden Kunststoffasern ausgebildet, wobei die beiden Maschenwaren 3a und 3b aus leitfähigen Fasern, zum Beispiel Kohlenstofffasern, ausgebildet sind und die jeweiligen Maschen einen Querschnitt aufweisen, der größer ist als das Größtkorn des Mörtels, der diejenige Mörtelschicht 4 ausbildet, in welcher das vorkonfektionierte Elektrodenpaar 3 eingebettet ist. Die Maschenwaren und das Abstandsgewirk bilden hier eine Bauteileinheit.

Alternativ können natürlich auch Drahtgitter als Elektrodenelement zum Einsatz kommen, insbesondere solche die durch nichtleitende Abstandshalter bereits auf festem Abstand gehalten werden und ein vorkonfektioniertes Bauteil bilden.

Die Erfindung kann demnach hier vorsehen, auf einer Untergrundkonstruktion, wie hier der Fahrbahn 1 eine Mörtelschicht aufzutragen, in diese Mörtelschicht, insbesondere quer zur Fahrbahnrichtung, ein vorkonfektioniertes Elektrodenelement 3 mit wenigstens einem Elektrodenpaar einzulegen, das Elektrodenelement 3 und dessen innere Hohlräume durch den Mörtel aufzufüllen und mit Mörtel zu überdecken, so dass sich insgesamt eine Mörtelschicht 4 ergibt, in welcher das vorkonfektionierte Elektrodenpaar eingeschlossen ist.

Hier ist der Mörtel für die Mörtelschicht 4 derart ausgewählt, dass dessen elektrischer Widerstand abhängig ist von der Feuchte des Mörtels. Der Mörtel bildet somit eine elektrolytisch wirksame Schicht im Sinn der Erfindung.

Es kann so durch eine Widerstandsmessung zwischen den Maschenwaren 3 a und 3b, die hier als Elektrodenflächen mit offener Oberfläche dienen, festgestellt werden, ob in die Mörtelschicht 4 Feuchtigkeit eingedrungen ist.

Unten an die Mörtelschicht 4 schließt die Untergrundkonstruktion 1 an, wobei oberhalb der Mörtelschicht 4 und somit oberhalb des erfindungsgemäßen Systems ein weiterer Fahrbahnaufbau 5 von grundsätzlich beliebiger Art anschließen kann. Hieraus ergibt sich, dass das erfindungsgemäße System Teil eines Gesamtaufbaus wie zum Beispiel einer Straße, einer Brücke oder auch eines sonstigen Bauwerkes bilden kann.

Die beiden Elektrodenflächen, die durch die Maschenwaren 3a und 3b gebildet werden, sind hier zum Zweck der Überwachung an eine Messvorrichtung angeschlossen, um denjenigen Flächenbereich der Untergrundkonstruktion, der durch ein Elektrodenpaar 3 überdeckt ist, auf Feuchte hin zu überwachen beziehungsweise durch Anschalten einer Spannung zum Schutz einer Diffusionsbarriere aufzubauen.

Die Figur 1 zeigt weiterhin, dass aufgrund begrenzter Fertigungsbreiten B von Elektrodenpaar-Elementen 3 es vorgesehen sein kann, ein jeweiliges Elektrodenelement 3 quer über die gesamte Fahrbahnbreite auszulegen und in Fahrbahnlängserstreckungsrichtung, das heißt in vorgesehener Fahrtrichtung, mehrere solche Elektrodenelemente hintereinander anzuordnen, so dass großflächig eine Fahrbahn insgesamt und grundsätzlich in beliebiger Länge überwacht werden kann.

Jedes einzelne Elektrodenpaar-Element 3, das hier aus dem mit Abstandsgewirk 3c verbundenen Maschenwaren 3a und 3b besteht, bildet einen eigenen überwachbaren Flächenbereich, der jeweils individuell für sich mit einer Messvorrichtung verbunden sein kann. Es kann hier auch vorgesehen sein, mehrere Elektrodenpaar-Elemente 3 mit derselben Messvorrichtung zu verbinden, wobei diese Messvorrichtung jedoch für jedes Elektrodenpaar individuell eine Widerstandsmessung vornimmt.

Auch kann es hier vorgesehen sein, die unteren oder die oberen Elektroden untereinander alle zu verschalten, da sich dennoch eine Flächenauflösung durch die jeweils zugeordnete und individuell verschaltete gegenüberliegende Elektrode ergibt.

Nicht visualisiert, jedoch vorgesehen sein kann es, dass bei Unterschreiten eines Widerstandsgrenzwertes bei einem Elektrodenpaar 3, dieses Unterschreiten visualisiert, akustisch oder durch Telekommunikation angezeigt wird. Es kann demnach vorgesehen sein, dass eine Messvorrichtung Sendemittel umfasst, um beispielsweise über Mobilfunk eine Warnmeldung über den festgestellten Feuchteintritt an eine Leitstelle oder sonstige Überwachungsstelle zu übermitteln.

So, wie hier die Möglichkeit gegeben ist, die Elektroden, beziehungsweise Maschenwaren 3a und 3b, mit einer Messvorrichtung zu verbinden, um den Widerstand zwischen diesen Elektroden zu messen, kann es ebenso vorgesehen sein, zum Schutz und insbesondere zum Aufbau einer Diffusionsbarriere und/oder kathodischen Korrosionsschutzes zwischen den Elektrodenflächen, beziehungsweise Maschenwaren 3a und 3b, eine Spannung an diese Elektrodenflächen anzulegen.

Je nach Richtung des hierdurch aufgebauten elektrischen Feldes, wird eine Diffusionsbarriere für entweder positiv oder negativ geladene Ionen ausgebildet, so dass beispielsweise eine Feldrichtung beziehungsweise Spannungspolarität gewählt werden kann, um eine Diffusionsbarriere für Chloridionen aufzubauen, da voraussichtlich hauptsächlich diese Ionenart durch Tausalzeintrag auf einer Fahrbahnoberfläche auftreten wird.

Aufgrund des Flexibilität eines Elektrodenpaares 3 wegen des Aufbaus als mit Abstandsgewirk verbundene Maschenware, kann ein Herstellungsverfahren hier vorsehen, dass quer zur Fahrbahnrichtung solche vorkonfektionierten Elektrodenelemente in ein Mörtelbett abgerollt werden oder unmittelbar auf die trockene Untergrundkonstruktion 1 abgerollt werden und sodann durch Auftragen von Mörtel in die Mörtelschicht 4 eingebettet werden. Es bildet sich so auf der Untergrundkonstruktion das erfindungsgemäße System, das sodann mit weiteren Aufbauten, z.B. einem Fahrbahnbelag belegt werden kann.

Das erfindungsgemäße System bildet somit sodann für die Zukunft ein untrennbar mit dem Gesamtbauwerk verbundenes Element, das fortwährend für die Überwachung oder zum Schutz der Untergrundkonstruktion verwendet werden kann.

## Patentansprüche

1. System zur Überwachung eines Untergrundes (1) hinsichtlich Schäden und/oder zum Schutz eines Untergrundes (1) vor Schäden, welches
a. eine elektrolytisch wirksame Schicht (4) umfasst, die einen feuchtigkeitsabhängigen elektrischen Widerstand aufweist und
b. wenigstens ein Elektrodenpaar aufweist, dessen beabstandete Elektroden (3a, 3b) über die elektrolytisch wirksame Schicht (4) miteinander verbunden sind, und
eine Messvorrichtung umfasst, mittels welcher eine Eigenschaft, der elektrolytisch wirksamen Schicht (4) oder der Elektroden (3a, 3b) mithilfe von Elektroden (3a, 3b) wenigstens eines Elektrodenpaares messbar ist und/oder eine Steuervorrichtung umfasst mittels der eine elektrische Spannung an die Elektroden (3a, 3b) wenigstens eines Elektrodenpaares anlegbar ist, **dadurch gekennzeichnet, dass** die elektrolytisch wirksame Schicht ausgebildet ist als zementöse ausgehärtete Schicht und wenigstens eine der Elektroden (3a, 3b) des wenigstens einen Elektrodenpaares durch eine Maschen-/Mattenware aus leitfähigen Fasern als eine flächige Elektrode in der zementös ausgehärteten, elektrolytisch wirksamen Schicht (4) ausgebildet ist, wobei in der Elektrode Durchgänge vorhanden sind, die einen Querschnitt aufweisen, der größer ist als die größten Feststoffanteile im Schichtmaterial und sich die Feststoffanteile durch die Durchgänge hindurch erstrecken.

2. System nach Anspruch 1, **dadurch gekennzeichnet, dass** die als Maschen-/Mattenware aus leitfähigen Fasern ausgebildeten Elektroden mehrfach wiederholt in wenigstens einer Erstreckungsrichtung des Untergrundes (1) nebeneinander gelegt sind.

3. System nach einem der vorherigen Ansprüche, **dadurch gekennzeichnet, dass** die Eigenschaft, insbesondere die elektrische Größe eine der folgenden ist:
a. der elektrische Widerstand;
b. die elektrische Impedanz;
c. eine Spannung, die zwischen zwei Elektroden anliegt;
d. ein Strom, der über die Elektroden und die elektrolytisch wirksame Schicht fließt; die Dielektrizität der elektrolytisch wirksamen Schicht.

4. System nach Anspruch 3, **dadurch gekennzeichnet, dass** die Eigenschaft eine solche ist, die aus einer elektrischen Größe nach Anspruch 3 bestimmt ist, insbesondere die Eigenschaft eine der folgenden ist:
a. die chemische Zusammensetzung der elektrolytisch wirksamen Schicht, insbesondere des Elektrolyten dieser Schicht;
b. der pH-Wert der elektrolytisch wirksamen Schicht;
c. die Temperatur der elektrolytisch wirksamen Schicht.

5. System nach einem der vorherigen Ansprüche, **dadurch gekennzeichnet, dass** der Untergrund ausgebildet ist durch wenigstens einen der folgenden:
a. Erdreich;
b. eine Deponie;
c. eine Betonunterkonstruktion, insbesondere eine stahlbewehrte Unterkonstruktion, bevorzugt eine Straße oder Brücke;
d. Metallische Konstruktion.

6. System nach einem der vorherigen Ansprüche, **dadurch gekennzeichnet, dass** die zementöse ausgehärtete elektrolytisch wirksame Schicht ausgebildet ist durch Mörtel oder Beton.

7. System nach einem der vorherigen Ansprüche, **dadurch gekennzeichnet, dass** es mittels der elektrolytisch wirksamen Schicht, insbesondere einem Mörtel oder Beton am Untergrund befestigt ist.

8. System nach einem der vorherigen Ansprüche, **dadurch gekennzeichnet, dass** eine erste Elektrode eines Elektrodenpaares durch die flächige Elektrode in der elektrolytisch wirksamen Schicht und eine zweite Elektrode eines Elektrodenpaares durch den Untergrund oder ein darin befindliches elektrisch leitfähiges Element ausgebildet ist, insbesondere durch eine elektrisch leitfähige Bewehrung einer Stahlbetonunterkonstruktion gebildet ist.

9. System nach einem der vorherigen Ansprüche, **dadurch gekennzeichnet, dass** eine erste Elektrode (3a) eines Elektrodenpaares durch die flächige Elektrode in der elektrolytisch wirksamen Schicht (4) und eine zweite Elektrode (3b) eines Elektrodenpaares durch eine weitere, insbesondere ebenfalls flächige Elektrode in der elektrolytisch wirksamen Schicht (4) gebildet ist, insbesondere wobei die beiden Elektroden (3a, 3b) in der elektrolytisch wirksamen Schicht flächengleich sind, insbesondere in Abstandsrichtung fluchtend hintereinander liegen.

10. System nach einem der vorherigen Ansprüche, **dadurch gekennzeichnet, dass** ein Elektrodenpaar ausgebildet ist als zwei mit Abstandhaltern (3c) auf Abstand gehaltene flächige Elektroden (3a, 3b), wobei die flächigen Elektroden (3a, 3b) aus je einer Matten- oder Maschenware aus leitfähigen Fasern, insbesondere Kohlenstofffasern ausgebildet sind, insbesondere wobei die beiden Lagen der Maschen-/Mattenware durch ein Gewirk (3c) nichtleitender Fasern, insbesondere Kunststofffasern auf Abstand gehalten sind.

11. System nach einem der vorherigen Ansprüche, **dadurch gekennzeichnet, dass** jedes Elektrodenelement (3a, 3b) oder Paar einen individuell überwachbaren und/oder schützbaren Bereich bildet.

12. Verfahren zur Herstellung eines Systems zur Überwachung und/oder zum Schützen eines Untergrundes, **dadurch gekennzeichnet, dass** wenigstens ein flächiges Elektrodenelement (3a) oder wenigstens ein Paar aus zwei flächigen und beabstandeten Elektrodenelementen (3a, 3b), jeweils umfassend eine Maschen-/Mattenware aus leitfähigen Fasern auf einem Untergrund (1), ausgelegt wird, wobei in dem wenigstens einen Elektrodenelement Durchgänge vorhanden sind, die einen Querschnitt aufweisen, der größer ist als die größten Feststoffanteile im Schichtmaterial und das wenigstens eine Elektrodenelement mit einer zementös aushärtbaren elektrisch leitfähigen Schicht (4), deren Leitfähigkeit von der Feuchtigkeit der Schicht abhängt, umgeben wird, deren Feststoffanteile sich durch die Durchgänge hindurch erstrecken, so dass sich eine Schicht ausbildet, in welcher wenigstens ein flächiges Elektrodenelement (3a, 3b) eingebettet ist und die Elektroden (3a, 3b) mit einer Messvorrichtung zum Messen einer Eigenschaft zwischen zwei Elektroden (3a, 3b) und/oder mit einer Steuervorrichtung zum Anlegen einer Spannung zwischen zwei Elektroden (3a 3b) verbunden wird.

13. Verfahren nach Anspruch 12, **dadurch gekennzeichnet, dass** eine Maschen-/Mattenware aus leitfähigen Fasern auf dem Untergrund (1) abgerollt wird.

14. Verfahren nach Anspruch 12 oder 13, **dadurch gekennzeichnet, dass** das Auslegen oder Abrollen mehrfach wiederholt nebeneinander in wenigstens einer Erstreckungsrichtung des Untergrundes (1) erfolgt.

15. Verfahren zur Überwachung eines Untergrundes hinsichtlich Schäden, insbesondere Feuchteschäden, **dadurch gekennzeichnet, dass** zwischen wenigstens zwei Elektroden (3a, 3b) eines Systems nach einem der vorherigen Ansprüche 1 bis 10 ein Wert einer Eigenschaft des Systems, insbesondere einer elektrischen Größe des Systems, bevorzugt ein Wert für den elektrischen Widerstand zwischen den Elektroden (3a, 3b) gemessen und mit wenigstens einem Vergleichswert verglichen wird, wobei bei einem Unterschreiten oder Überschreitung des wenigstens einen Vergleichswertes, insbesondere aufgrund ansteigender Feuchtigkeit zwischen den wenigstens zwei Elektroden (3a, 3b) eine Signalisierung erfolgt.

16. Verfahren zum Schutz eines Untergrundes vor Schäden, insbesondere Feuchteschäden und/oder Schäden infolge des Eindringens von Schadstoffen, **dadurch gekennzeichnet, dass** zwischen wenigstens zwei Elektroden (3a, 3b) eines Systems nach einem der vorherigen Ansprüche 1 bis 10 mittels einer Steuervorrichtung eine Spannungsdifferenz angelegt wird, insbesondere zur Erzeugung einer Diffusionsbarriere oder eines kathodischen Korrosionsschutzes, insbesondere wobei das Anlegen der Spannung, bevorzugt automatisch erfolgt nach Signalisierung des Unterschreitens oder Überschreitens des wenigstens einen Vergleichswertes zwischen den Elektroden gemäß Anspruch 15.

## Claims

1. System for monitoring a substratum (1) in respect of damage and/or for protecting a substratum (1) against damage, which comprises
a. an electrolytically active layer (4) which has a moisture-dependent electrical resistance and
b. at least one electrode pair whose spaced electrodes (3a, 3b) are connected to one another via the electrolytically active layer (4), and comprises a measuring device by means of which a property of the electrolytically active layer (4) or of the electrodes (3a, 3b) can be measured by means of electrodes (3a, 3b) of at least one electrode pair and/or comprises a control device by means of which an electric potential can be applied to the electrodes (3a, 3b) of at least one electrode pair, **characterized in that** the electrolytically active layer is configured as cement-based cured layer and at least one of the electrodes (3a, 3b) of the at least one electrode pair is formed by a mesh/mat of conductive fibres as sheet-like electrode in the cement-based cured, electrolytically active layer (4), where passages which have a cross section which is greater than the largest solid particles in the layer material are present in the electrode and the solid particles extend through the passages.

2. System according to Claim 1, **characterized in that** the electrodes configured as mesh/mat of conductive fibres are repeatedly laid next to one another in at least one extension direction of the substratum (1) .

3. System according to either of the preceding claims, **characterized in that** the property, in particular the electrical parameter, is one of the following:
a. the electrical resistance;
b. the electrical impedance;
c. an electric potential prevailing between two electrodes;
d. a current which flows through the electrodes and the electrolytically active layer; the dielectric property of the electrolytically active layer.

4. System according to Claim 3, **characterized in that** the property is a property which is determined from an electrical parameter according to Claim 3, in particular the property is one of the following:
a. the chemical composition of the electrolytically active layer, in particular of the electrolyte of this layer;
b. the pH of the electrolytically active layer;
c. the temperature of the electrolytically active layer.

5. System according to any of the preceding claims, **characterized in that** the substratum is formed by at least one of the following:
a. earth;
b. a landfill;
c. a concrete subconstruction, in particular a steel-reinforced subconstruction, preferably a road or bridge;
d. metallic construction.

6. System according to any of the preceding claims, **characterized in that** the cement-based cured electrolytically active layer is formed by mortar or concrete.

7. System according to any of the preceding claims, **characterized in that** it is fastened to the substratum by means of the electrolytically active layer, in particular a mortar or concrete.

8. System according to any of the preceding claims, **characterized in that** a first electrode of an electrode pair is formed by the sheet-like electrode in the electrolytically active layer and a second electrode of an electrode pair is formed by the substratum or an electrically conductive element present therein, in particular by electrically conductive reinforcement of a reinforced concrete subconstruction.

9. System according to any of the preceding claims, **characterized in that** a first electrode (3a) of an electrode pair is formed by the sheet-like electrode in the electrolytically active layer (4) and a second electrode (3b) of an electrode pair is formed by a further, in particular likewise sheet-like, electrode in the electrolytically active layer (4), in particular with the two electrodes (3a, 3b) in the electrolytically active layer having the same area, in particular being flush behind one another in the spacing direction.

10. System according to any of the preceding claims, **characterized in that** an electrode pair is configured as two sheet-like electrodes (3a, 3b) held at a distance from one another by spacers (3c), where the sheet-like electrodes (3a, 3b) are in each case made of a mat or mesh of conductive fibres, in particular carbon fibres, in particular with the two layers of the mesh/mat being kept at a distance from one another by a formed-loop knit (3c) of nonconductive fibres, in particular polymer fibres.

11. System according to any of the preceding claims, **characterized in that** each electrode element (3a, 3b) or pair forms an individual monitorable and/or protectable region.

12. Method for producing a system for monitoring and/or protecting a substratum, **characterized in that** at least one sheet-like electrode element (3a) or at least one pair of two sheet-like and spaced electrode elements (3a, 3b), in each case comprising a mesh/mat of conductive fibres, is placed on a substratum (1), where passages which have a cross section which is greater than the largest solid particles in the layer material are present in the at least one electrode element and the at least one electrode element is surrounded by a cement-based curable electrically conductive layer (4) whose conductivity depends on the moisture content of the layer and whose solid particles extend through the passages, so as to form a layer in which at least one sheet-like electrode element (3a, 3b) is embedded, and the electrodes (3a, 3b) are connected to a measuring device for measuring a property between two electrodes (3a, 3b) and/or to a control device for applying an electric potential between two electrodes (3a, 3b).

13. Method according to Claim 12, **characterized in that** a mesh/mat of conductive fibres is rolled off onto the substratum (1).

14. Method according to Claim 12 or 13, **characterized in that** the laying or rolling-off is carried out repeatedly next to one another in at least one extension direction of the substratum (1).

15. Method for monitoring a substratum in respect of damage, in particular moisture damage, **characterized in that** a value of a property of the system, in particular an electrical parameter of the system, preferably a value for the electrical resistance between the electrodes (3a, 3b), is measured between at least two electrodes (3a, 3b) of a system according to any of Claims 1 to 10 and is compared with at least one comparative value, with signalling occurring in the case of the measured value going below or above the at least one comparative value, in particular as a result of an increasing moisture content between the at least two electrodes (3a, 3b).

16. Method for protecting a substratum against damage, in particular moisture damage and/or damage resulting from the intrusion of pollutants, **characterized in that** a potential difference is applied by means of a control device between at least two electrodes (3a, 3b) of a system according to any of Claims 1 to 10, in particular so as to produce a diffusion barrier or cathodic corrosion protection, in particular with the application of the electric potential preferably occurring automatically after signalling of the fact that the measured value has gone below or above the at least one comparative value between the electrodes according to Claim 15.

## Revendications

1. Système de surveillance d'un sous-sol (1) pour déterminer ses dommages et/ou pour la protection d'un sous-sol (1) contre des dommages, qui comprend
a. une couche électrolytiquement active (4) qui présente une résistance électrique dépendant de l'humidité et
b. qui présente au moins une paire d'électrodes dont les électrodes distantes (3a, 3b) sont reliées les unes aux autres par l'intermédiaire de la couche électrolytiquement active (4) et
qui comprend un dispositif de mesure au moyen duquel une propriété de la couche électrolytiquement active (4) ou des électrodes (3a, 3b) peut être mesurée à l'aide des électrodes (3a, 3b) d'au moins une paire d'électrodes et/ou un dispositif de commande au moyen duquel une tension électrique peut être appliquée aux électrodes (3a, 3b) d'au moins une paire d'électrodes,
**caractérisé en ce que** la couche électrolytiquement active est réalisée sous la forme d'une couche durcie à base de ciment et au moins l'une des électrodes (3a, 3b) de ladite au moins une paire d'électrodes est constituée d'un tissu maillé/mat de fibres conductrices sous la forme d'une électrode plate dans la couche électrolytiquement active (4) durcie à base de ciment, dans lequel des passages, dont la section transversale est plus grande que les plus grandes parties solides dans le matériau de la couche, sont ménagés dans l'électrode et les parties solides s'étendent à travers les passages.

2. Système selon la revendication 1, **caractérisé en ce que** les électrodes, qui sont réalisées sous la forme de tissu maillé/mat de fibres conductrices, sont posées plusieurs fois de manière répétée côte à côte dans au moins une direction d'extension du sous-sol (1).

3. Système selon l'une des revendications précédentes, **caractérisé en ce que** la propriété, en particulier la grandeur électrique, est l'une des suivantes :
a. la résistance électrique ;
b. l'impédance électrique ;
c. une tension appliquée entre deux électrodes ;
d. un courant passant dans les électrodes et la couche électrolytiquement active ; le coefficient diélectrique de la couche électrolytiquement active.

4. Système selon la revendication 3, **caractérisé en ce que** la propriété est une propriété qui est déterminée à partir d'une grandeur électrique selon la revendication 3, la propriété étant en particulier l'une des suivantes :
a. la composition chimique de la couche électrolytiquement active, en particulier de l'électrolyte de ladite couche ;
b. le pH de la couche électrolytiquement active ;
c. la température de la couche électrolytiquement active.

5. Système selon l'une des revendications précédentes, **caractérisé en ce que** le sous-sol est constitué par au moins l'un des éléments suivants :
a. le sol ;
b. un site d'enfouissement ;
c. une sous-structure en béton, en particulier une sous-structure renforcée par de l'acier, de préférence une route ou un pont ;
d. une construction métallique.

6. Système selon l'une des revendications précédentes, **caractérisé en ce que** la couche électrolytiquement active durcie à base de ciment est formée par du mortier ou du béton.

7. Système selon l'une des revendications précédentes, **caractérisé en ce qu'**il est fixé au sous-sol au moyen de la couche électrolytiquement active, en particulier par du mortier ou du béton.

8. Système selon l'une des revendications précédentes, **caractérisé en ce qu'**une première électrode d'une paire d'électrodes est constituée par l'électrode plate dans la couche électrolytiquement active et **en ce qu'**une deuxième électrode d'une paire d'électrodes est constituée par le sous-sol ou un élément électriquement conducteur qui s'y trouve, en particulier par une armature électriquement conductrice d'une sous-structure en béton armé.

9. Système selon l'une des revendications précédentes, **caractérisé en ce qu'**une première électrode (3a) d'une paire d'électrodes est formée par l'électrode plate dans la couche électrolytiquement active (4) et **en ce qu'**une deuxième électrode (3b) d'une paire d'électrodes est formée par une autre électrode, en particulier également plate, dans la couche électrolytiquement active (4), en particulier dans lequel les deux électrodes (3a, 3b) de la couche électrolytiquement active présentent la même superficie, en particulier sont alignées l'une derrière l'autre dans la direction de la distance.

10. Système selon l'une des revendications précédentes, **caractérisé en ce qu'**une paire d'électrodes est réalisée sous la forme de deux électrodes plates (3a, 3b) maintenues à distance par des entretoises (3c), dans lequel les électrodes plates (3a, 3b) sont chacune constituées d'un tissu maillé ou d'un mat de fibres conductrices, en particulier de fibres de carbone, en particulier dans lequel les deux couches du tissu maillé/mat sont maintenues à distance par un tricot (3c) de fibres non conductrices, en particulier de fibres de plastique.

11. Système selon l'une des revendications précédentes, **caractérisé en ce que** chaque élément d'électrode (3a, 3b) ou paire d'électrodes forme une zone pouvant être surveillée et/ou pouvant être protégée individuellement.

12. Procédé de fabrication d'un système de surveillance et/ou de protection d'un sous-sol, **caractérisé en ce qu'**on pose au moins un élément d'électrode plat (3a) ou au moins une paire de deux éléments d'électrodes plats et distants (3a, 3b), comprenant chacun un tissu maillé/mat de fibres conductrices sur un sous-sol (1), dans lequel des passages sont présents dans ledit au moins un élément d'électrode, lesquels passages présentent une section transversale qui est plus grande que les plus grandes parties solides dans le matériau de la couche, et ledit au moins un élément d'électrode est entouré d'une couche électriquement conductrice (4) durcissable à base de ciment, dont la conductivité dépend de l'humidité de la couche, dont les parties solides s'étendent à travers les passages, de sorte qu'il se forme une couche dans laquelle est noyé au moins un élément d'électrode plat (3a, 3b), et les électrodes (3a, 3b) sont reliées à un dispositif de mesure destiné à mesurer une propriété entre deux électrodes (3a, 3b) et/ou à un dispositif de commande destiné à appliquer une tension entre deux électrodes (3a, 3b).

13. Procédé selon la revendication 12, **caractérisé en ce qu'**un tissu maillé/mat de fibres conductrices est déroulé sur le sous-sol (1).

14. Procédé selon la revendication 12 ou 13, **caractérisé en ce que** la pose ou le déroulement est effectué plusieurs fois de manière répétée côte à côte dans au moins une direction d'extension du sous-sol (1).

15. Procédé de surveillance d'un sous-sol pour détecter des dommages, en particulier des dommages dus à l'humidité, **caractérisé en ce qu'**entre au moins deux électrodes (3a, 3b) d'un système selon l'une des revendications 1 à 10 précédentes une valeur d'une propriété du système, en particulier une grandeur électrique du système, de préférence une valeur de la résistance électrique entre les électrodes (3a, 3b), est mesurée et comparée à au moins une valeur de comparaison, dans lequel une signalisation est effectuée en cas de dépassement inférieur ou supérieur de ladite au moins une valeur de comparaison, en particulier du fait d'une augmentation de l'humidité entre lesdites au moins deux électrodes (3a, 3b).

16. Procédé de protection d'un sous-sol contre des dommages, en particulier des dommages dus à l'humidité et/ou des dommages dus à la pénétration de polluants, **caractérisé en ce qu'**une différence de tension est appliquée entre au moins deux électrodes (3a, 3b) d'un système selon l'une des revendications 1 à 10 précédentes au moyen d'un dispositif de commande, en particulier pour générer une barrière de diffusion ou une protection cathodique contre la corrosion, en particulier dans lequel l'application de la tension est effectuée, de préférence automatiquement, après signalisation du dépassement inférieur ou supérieur de ladite au moins une valeur de comparaison entre les électrodes selon la revendication 15.
